(19) Europäisches Patentamt
European Patent Office
Office européen des brevets

(11) EP 2 111 852 A2

(12) **EUROPÄISCHE PATENTANMELDUNG**

(43) Veröffentlichungstag:
**28.10.2009 Patentblatt 2009/44**

(51) Int Cl.:
***A61K 8/41*** *(2006.01)* ***A61Q 5/12*** *(2006.01)*

(21) Anmeldenummer: **09157680.1**

(22) Anmeldetag: **09.04.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **21.04.2008 DE 102008020044**

(71) Anmelder: **Henkel AG & Co. KGaA**
**40589 Düsseldorf (DE)**

(72) Erfinder: **Battermann, Marlene**
**21271 Asendorf (DE)**

# (54) Pflegendes Haarbehandlungsverfahren

(57) Ein Haarbehandlungsverfahren bereitzustellen, das die keratinischen Fasern in besonderer Weise pflegt und die Eigenschaften des behandelten Haares, insbesondere Naß- und Trockenkämmbarkeit sowie den Griff des nassen und trockenen Haares verbessert, wobei in dessen Rahmen kostengünstige und lagerstabile wäßrige Mittel eingesetzt werden können, umfaßt die Schritte a) Auftragen einer Haarbehandlungszusammensetzung, die mindestens eine kationische Verbindung der Formel (I)

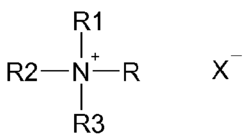

(I),

in der
- die Reste R, R1, R2 unabhängig voneinander für einen gegebenenfalls substituierten Alkyl- oder Alkenyl- oder Acylrest stehen, wobei
- R1 und R2 miteinander einen Ring bilden können,
- R3 für $-CH_3$ oder $-CH_2CH_3$ steht, und
- X für ein Anion aus der Gruppe Chlorid, Bromid, Methosulfat steht,

mindestens einen Pflegestoff und mindestens 30 Gew.-% Wasser enthält, auf trockenes oder handtuchtrockenes oder nasses Haar, b) Abdeckung der Haare, daß keine Komponente der Haarbehandlungszusammensetzung entweichen kann, c) Einwirkenlassen der Zusammensetzung über einen Zeitraum von 30 bis 600 Sekunden, d) Abnehmen der Abdeckung und Auswaschen der Haarbehandlungszusammensetzung.

EP 2 111 852 A2

## Beschreibung

Die Erfindung betrifft ein Verfahren zur Haarbehandlung.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate wirken im allgemeinen bevorzugt an der Haaroberfläche. So sind Wirkstoffe bekannt, welche dem Haar Glanz, Halt, Fülle, bessere Naß- oder Trockenkämmbarkeiten verleihen oder dem Spliß vorbeugen.

Die bekannten Wirkstoffe können jedoch nicht alle Bedürfnisse in ausreichendem Maße abdecken. Jenseits des bestehenden Bedarfes nach neuen Wirkstoffen bzw. Wirkstoffkombinationen für kosmetische Mittel mit guten pflegenden Eigenschaften und guter biologischer Abbaubarkeit besteht daher zusätzlich ein Bedarf nach neuen Applikationsformen und - bedingungen um bekannte Wirkstoffe optimal zur Wirkung zu bringen.

Der vorliegenden Erfindung lag die Aufgabe zugrunde, ein Haarbehandlungsverfahren bereitzustellen, das die keratinischen Fasern in besonderer Weise pflegt. Dabei sollten die Eigenschaften des behandelten Haares, insbesondere Naß- und Trockenkämmbarkeit sowie den Griff des nassen und trockenen Haares verbessert werden. Eine weitere Aufgabe besteht darin, das angegriffene Haar in seiner gesamten Struktur wieder zu stärken und zu regenerieren. Dies wird als Strukturanteneffekt bezeichnet.

Es wurde nun gefunden, daß sich die pflegende Wirkung von bestimmten Haarbehandlungsmitteln mit speziellen steigern läßt, wenn definierte Applikationsbedingungen eingehalten werden.

Die EP 1 147 762 (Kao) offenbart ein nicht-therapeutisches Verfahren zur Behandlung von Haar, umfassend das Auftragen einer Haarbehandlungszusammensetzung, die mindestens ein öliges Agens, das aus der Gruppe ausgewählt ist, die aus Esterölen, Triglyceridölen, Kohlenwasserstoffölen, pflanzlichen Ölen, tierischen Ölen, Cetanol, Myristylalkohol, Stearylalkohol, Behenylalkohol, 2-Octyldodecanol und einer Mischung hiervon besteht, und ein Lösungsmittel umfasst und einen Wassergehalt von 20 % oder weniger aufweist, auf trockenes Haar, das Erwärmen des Haares auf eine Temperatur von 38 bis 65°C für eine vorgeschriebene Dauer, wobei das Haar so bedeckt wird, dass keine Komponente der Haarbehandlungszusammensetzung entweichen kann, und das Auswaschen der Haarbehandlungszusammensetzung.

Diese Schrift lehrt, daß Öle bei Wärme besser in das Haar penetrieren und länger dort verbleiben, wenn sie aus wasserarmen Formulierungen bei erhöhter Temperatur aufgebracht werden. Allerdings führen diese Öle zu einer Beschwerung des Haares und teilweise zu einem unerwünschtem fettigen Glanz. Zusätzlich sind wasserarme Formulierungen aufwendiger und teurer in der Herstellung, weniger lagerstabil und werden vom Verbraucher oft überdosiert, was zu einer Verstärkung der vorstehend genannten Effekte und zu einem hohen Kostenaufwand auf Verbraucherseite führt.

Eine weitere Aufgabe der vorliegenden Erfindung bestand daher auch darin, ein Verfahren bereitzustellen, in dessen Rahmen kostengünstige und lagerstabile wäßrige Mittel eingesetzt werden können.

Gegenstand der vorliegenden Erfindung ist ein Verfahren zur kosmetischen Behandlung von Haar, umfassend die Schritte

a) Auftragen einer Haarbehandlungszusammensetzung, die

i. mindestens eine kationische Verbindung der Formel (I)

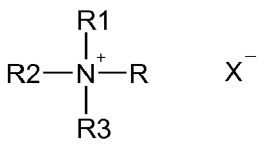

(I),

in der

- die Reste R, R1, R2 unabhängig voneinander für einen gegebenenfalls substituierten Alkyl- oder Alkenyl- oder Acylrest stehen, wobei
- R1 und R2 miteinander einen Ring bilden können,
- R3 für-$CH_3$ oder -$CH_2CH_3$ steht, und
- $X^-$ für ein Anion aus der Gruppe Chlorid, Bromid, Methosulfat steht,

ii. mindestens einen Pflegestoff,
iii. mindestens 30 Gew.-% Wasser

enthält,
auf trockenes oder handtuchtrockenes oder nasses Haar,
b) Abdeckung der Haare, daß keine Komponente der Haarbehandlungszusammensetzung entweichen kann
c) Einwirkenlassen der Zusammensetzung über einen Zeitraum von 30 bis 600 Sekunden,
d) Abnehmen der Abdeckung und Auswaschen der Haarbehandlungszusammensetzung.

Ein wesentliches Element der vorliegenden Erfindung besteht im Einsatz einer wäßrigen Pflegezusammensetzung, die mindestens eine kationische Verbindung, mindestens einen Pflegestoff und Wasser enthält. Bevorzugte erfindungsgemäße Verfahren setzen Zusammensetzungen ein, die mehr als 30 Gew.-% Wasser enthalten. Bevorzugte Verfahren nutzen Haarbehandlungszusammensetzungen, die - bezogen auf ihr Gewicht - mindestens 35 Gew.-%, vorzugsweise mindestens 40 Gew.-%, weiter bevorzugt mindestens 45 Gew.-%, noch weiter bevorzugt mindestens 50 Gew.-%, weiter bevorzugt mindestens 55 Gew.-% und insbesondere mindestens 60 Gew.-% Wasser enthalten. In besonders bevorzugten Verfahren werden Haarbehandlungszusammensetzungen eingesetzt, die - bezogen auf ihr Gewicht - mindestens 62,5 Gew.-%, vorzugsweise mindestens 65 Gew.-%, weiter bevorzugt mindestens 67,5 Gew.-%, noch weiter bevorzugt mindestens 70 Gew.-%, weiter bevorzugt mindestens 72,5 Gew.-% und insbesondere mindestens 80 Gew.-% Wasser enthalten.
Als zweiten wesentlichen Bestandteil enthalten die im erfindungsgemäßen Verfahren eingesetzten Haarbehandlungszusammensetzungen mindestens eine kationische Verbindung der Formel (I). Diese Verbindung(en) wird/ werden vorzugsweise innerhalb bestimmter Mengenbereiche eingesetzt.
Bevorzugte erfindungsgemäße Verfahren sind dadurch gekennzeichnet, daß das Haarbehandlungsmittel - bezogen uf sein Gewicht - 0,25 bis 10 Gew.-%, vorzugsweise 0,5 bis 7,5 Gew.-%, weiter bevorzugt 0,75 bis 5 Gew.-% und insbesondere 1 bis 3,5 Gew.-% kationische Verbindung(en) der Formel (I) enthält.
Eine bevorzugte Gruppe von Verbindungen, die im erfindungsgemäßen Verfahren eingesetzt werden kann, sind Verbindungen der Formel (I), in der die Reste R1, R2 und R3 für-$CH_3$ stehen und R einen langkettigen gesättigten oder ungesättigten Alkyl-, Alkenyl- oder Acylrest darstellt. Besonders bevorzugt ist unter diesen Verbindungen des Behenoyl-trimethylammoniumchlorid, das laut INCI als Behenoyl PG-Trimonium Chloride bezeichnet wird und unter der Handelsbezeichnung Quartamin® BTC erhältlich ist.
Die Auswahl der Reste R, R1, R2 und R3 in Formel (I) kann zu unterschiedlichen Verbindungen führen. Eine bevorzugte Gruppe von Verbindungen weist zwei Methylreste und zwei längerkettige Alkylreste auf. Bevorzugte erfindungsgemäße Verfahren sind demnach dadurch gekennzeichnet, daß die Haarbehandlungszusammensetzung mindestens eine kationische Verbindung der Formel (Ia) enthält

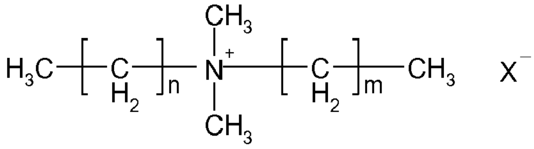

(Ia),

in der n und m unabhängig voneinander für ganze Zahlen zwischen 5 und 21 stehen, wobei bevorzugte Werte für n und

m die Zahlen 7, 9, 11, 13, 15 und 17 sind und bevorzugt n = m = 17 gilt und $X^-$ für ein Anion aus der Gruppe Chlorid, Bromid, Methosulfat steht.

Die Vertreter, in denen m = n gilt, sind besonders bevorzugt. Eine ganz besonders bevorzugte Verbindung der Formel (Ia) ist Distearyldimethylammoniumchlorid (DSDMAC, n = m =17, X = Cl).

Zusätzlich zu kationischen Verbindungen der Formel (Ia) oder an deren Stelle können die im erfindungsgemäßen Verfahren eingesetzten Mittel weitere kationische Verbindungen der Formel (I) enthalten. Hier haben sich die sogenannten "Esterquats" bewährt, d.h. Verbindungen der Formel (I), in denen der Rest R für einen Hydroxyalkylrest und R1 und R2 für ggf. substituierte Acylreste stehen.

Bevorzugte erfindungsgemäße Verfahren sind dadurch gekennzeichnet, daß die Haarbehandlungszusammensetzung mindestens eine kationische Verbindung der Formel (Ib) enthält

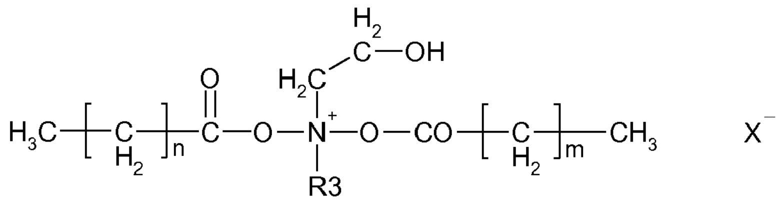

(Ib),

in der R3 für $-CH_3$ oder $-CH_2CH_3$ steht, n und m unabhängig voneinander für ganze Zahlen zwischen 4 und 20 stehen, wobei bevorzugte Werte für n und m die Zahlen 6, 8, 10, 12, 14 und 16 sind und bevorzugt n = m = 16 gilt und $X^-$ für ein Anion aus der Gruppe Chlorid, Bromid, Methosulfat steht.

Eine besonders bevorzugt im Rahmen des erfindungsgemäßen Verfahrens einsetzbare Verbindung ist die Verbindung mit zwei Stearoylresten, einem Ethyl- und einem Hydroxyethylrest. Diese wird nach INCI als Distearoylethyl Hydroxyethylmonium Methosulfate & Cetearyl Alcohol bezeichnet und ist unter dem Handelsnamen DEHYQUART® F 75 von der Fa. Cognis erhältlich.

Zusätzlich zu kationischen Verbindungen der Formeln (Ia) und/oder (Ib) oder an deren Stelle können die im erfindungsgemäßen Verfahren eingesetzten Mittel weitere kationische Verbindungen der Formel (I) enthalten. Hier haben sich die sogenannten "Imidazoliniumquats" bewährt, d.h. Verbindungen der Formel (I), in denen der Rest R für einen Amidoalkylrest steht und R1 und R2 miteinander einen N-substituierten Ring bilden.

Bevorzugte erfindungsgemäße Verfahren sind dadurch gekennzeichnet, daß die Haarbehandlungszusammensetzung mindestens eine kationische Verbindung der Formel (Ic) enthält

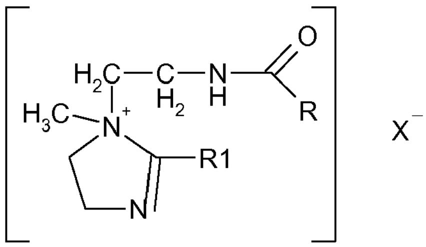

(Ic),

in der R für einen linearen Alkyl- oder Alkenylrest mit 7 bis 21 C-Atomen und R1 für -H oder $-CH_3$ oder $-CH_2CH_3$ oder $-CH(CH_3)_2$ oder $-CH_2CH_2CH_3$ und $X^-$ für ein Anion aus der Gruppe Chlorid, Bromid, Methosulfat steht.

Unter den Imidazolin-basierten quartären Verbindungen der Formel (I) und (Ic) im Besonderen, hat sich das nach INCI als Quaternium-87 bezeichnete und im Handel als VARISOFT® W 575 erhältliche Produkt als besonders geeignet erwiesen.

Als dritten Inhaltsstoff enthalten die im erfindungsgemäßen Verfahren eingesetzten Haarbehandlungsmittel mindestens einen Pflegestoff. Diese stammen bevorzugt aus bestimmten Gruppen: Besonders bevorzugte erfindungsgemäße Verfahren sind dadurch gekennzeichnet, daß die Haarbehandlungszusammensetzung mindestens einen Pflegestoff aus der Gruppe

i. L-Carnitin und/oder seiner Salze;
ii. Panthenol und/oder Panthothensäure;
iii. der 2-Furanone und/oder deren Derivate, insbesondere Pantolacton;

iv. Taurin und/oder seiner Salze;
v. Niacinamid;
vi. Ubichinon
vii. Ectoin;
viii. Allantoin;
ix. Extrakten von Echinacea

enthält.

Diese Pflegestoffe werden nachstehend beschrieben

L-Carnitin (IUPAC-Name(*R*)-(3-Carboxy-2-hydroxypropyl)- *N,N,N*-trimethylammoniumhydroxid), ist eine natürlich vorkommende, vitaminähnliche Substanz.

Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat.

Erfindungsgemäße bevorzugte Verfahren sind dadurch gekennzeichnet, daß die Haarbehandlungszusammensetzungen- bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Panthenol (IUPAC-Name: (+)-(R)-2,4-Dihydroxy-N-(3-hydroxypropyl)-3,3-dimethylbutyramid) wird im Körper zu Pantothensäure umgewandelt. Pantothensäure ist ein Vitamin aus der Gruppe der B-Vitamine (Vitamin B5).

Erfindungsgemäße bevorzugte Verfahren sind dadurch gekennzeichnet, daß die Haarbehandlungszusammensetzungen - bezogen auf ihr Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol (($\pm$)-2,4-Dihydroxy-*N*-(3-hydroxypropyl)-3,3-dimethylbutyramid) enthalten.

In erfindungsgemäß bevorzugten Verfahren enthalten die Haarbehandlungszusammensetzungen - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% mindestens eines 2-Furanonderivats der Formel (Fur-I) und/oder der Formel (Fur-II)

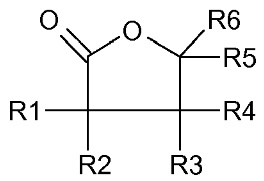 (Fur-I) 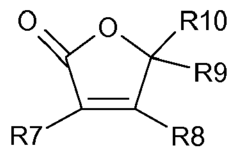 (Fur-II)

In einer besonders bevorzugten Ausführungsform der erfindungsgemäßen Lehre wird als Verbindung entsprechend der Formel (Fur-I) (R)-(-)-4-Hydroxymethyl-γ-butyrolacton und/oder D,L-4-Hydroxymethyl-γ-butyrolacton und/oder (S)-(+)-4-Hydroxymethyl-γ-butyrolacton und/oder R-(-)-2-Hydroxy-3,3-dimethyl-γ-butyrolacton und/oder D,L-2-Hydroxy-3,3-dimethyl-γ-butyrolacton und/oder S(+)-2-Hydroxy-3,3-dimethyl-γ-butyrolacton und/oder 4-Hydroxy-2,5-dimethyl-3(2H)-furanon und/oder Tetrahydro-5-oxo-2-furancarbonsäure und/oder Tetrahydro-5-oxo-2-furancarbonsäure, Na-Salz und/oder Tetrahydro-5-oxo-2-furancarbonsäure, K-Salz und/oder 2,5-Dihydro-5-methoxy-2-furanon und/oder Dihydro-3-hydroxy-4,4-dimethyl-2(3*H*)-furanon eingesetzt. In einer ganz besonders bevorzugten Ausführungsform der erfindungsgemäßen Lehre wird als Verbindung entsprechend der Formel (Fur-I) Dihydro-3-hydroxy-4,4-dimethyl-2(3H)-furanon eingesetzt.

Ein weiterer, bevorzugter einsetzbarer Pflegestoff, ist das Taurin. In erfindungsgemäß bevorzugten Verfahren enthalten die Haarbehandlungszusammensetzungen - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen sind Vitamine, Provitamine oder Vitaminvorstufen. Diese werden nachfolgend beschrieben:

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie

dessen Ester wie das Palmitat und das Acetat in Betracht. Die im erfindungsgemäßen Verfahren eingesetzten Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.

- Vitamin $B_1$ (Thiamin)
- Vitamin $B_2$ (Riboflavin)
- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin $B_5$ (Pantothensäure, Panthenol und Pantolacton). Im Rahmen dieser Gruppe wird bevorzugt das Panthenol und/oder Pantolacton eingesetzt (siehe weiter unten). Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin $B_5$-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin $B_6$ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere $\alpha$-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Verfahren bevorzugt, bei denen die Haarbehandlungszusammensetzungen - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-*N*-(3-hydroxypropyl)-3,3-dimethyl-butyramid, Provitamin $B_5$) und/oder Pantothensäure (Vitamin $B_3$, Vitamin $B_5$) und/oder Niacin, Niacinamid bzw. Nicotinamid (Vitamin $B_3$) und/oder L-Ascorbinsäure (Vitamin C) und/oder Thiamin (Vitamin $B_1$) und/oder Riboflavin (Vitamin $B_2$, Vitamin G) und/oder Biotin (Vitamin $B_7$, Vitamin H) und/oder Folsäure (Vitamin $B_9$, Vitamin $B_c$ oder Vitamin M) und/oder Vitamin $B_6$ und/oder Vitamin $B_{12}$ enthalten.

Es hat sich gezeigt, daß bestimmte Chinone eine besondere Eignung als Pflegestoffe besitzen. Als weitere Pflegestoffe können die im erfindungsgemäßen Verfahren eingesetzten Mittel daher 0,0001 bis 5 Gew.-% - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten

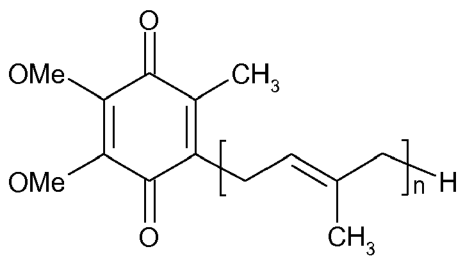

(Ubi),

in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die im erfindungsgemäßen Verfahren eingesetzten Mittel auch Plastochinone enthalten. Hier sind bevorzugte erfindungsgemäße Verfahren dadurch gekennzeichnet, daß die Haarbehandlungszusammensetzungen 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten

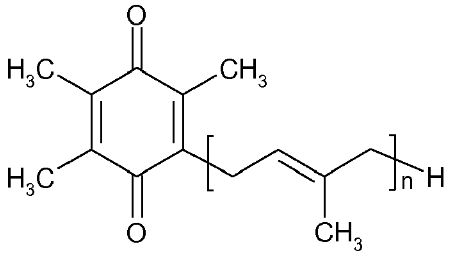

(Ubi-b)

in der n für Werte von 1 bis 20, vorzugsweise von 2 bis 15 und insbesondere für 5, 6, 7, 8, 9, 10 steht, wobei besonders bevorzugte Mittel Plastochinon PQ-9 enthalten.

Als weiteren Pflegestoff können die im erfindungsgemäßen Verfahren eingesetzten Mittel Ectoin enthalten. Ectoin ((4S)-2-Methyl-1,4,5,6-Tetrahydropyrimidin-4-Carbonsäure) ist ein Naturstoff. Erfindungsgemäß bevorzugte Verfahren sind dadurch gekennzeichnet, daß die Haarbehandlungszusammensetzungen - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Ein weiterer Pflegestoff ist Allantoin. Das Allantoin *(5-Ureidohydantoin, N*-(2,5-Dioxo-4-imidazolidinyl)-harnstoff) ist bei verschiedenen Tierarten, vor allem bei Säugetieren, neben der Harnsäure das Endprodukt des Abbaus von Nukleinsäuren, speziell von Purinbasen.

In erfindungsgemäß besonders bevorzugten Verfahren enthalten die Haarbehandlungszusammensetzungen - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% 5-Ureidohydantoin (Allantoin).

Als weitere Pflegestoffe eignen sich erfindungsgemäß Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Insbesondere bevorzugt sind Extrakte von Echinacea- oder Moringa-Pflanzen, wobei Echinacea-Extrakte besonders bevorzugt sind.

Erfindungsgemäß besonders bevorzugte Verfahren sind dadurch gekennzeichnet, daß die Haarbehandlungszusammensetzungen - bezogen auf ihr Gewicht - 0,001 bis 10 Gew.-%, insbesondere 0,01 bis 2 Gew.-% Wirkstoff, erhältlich aus Pflanzen der Gattung Echinacea, vorzugsweise aus Presssäften und Extrakten, die aus Echinacea purpurea gewonnen werden, enthalten.

Zur Verbesserung der Elastizität und Festigung der inneren Struktur der mittels des erfindungsgemäßen Verfahrens behandelter Haare können die hierin eingesetzten Mittel Purin und/oder Purinderivate als Pflege-Enhancer enthalten. Insbesondere die Kombination von Purin und/oder Purinderivaten mit Ubichinonen und/oder Plastochinonen als Pflege-Enhancer führt dazu, daß die mit entsprechenden Mitteln behandelten Haare unter anderem höhere Meßwerte bei der Differenzthermoanalyse und verbesserte Naß- und Trockenkämmbarkeiten zeigen.

Bevorzugte im erfindungsgemäßen Verfahren eingesetzte Mittel enthalten Purin und/oder Purinderivate in engeren Mengenbereichen. Hier sind erfindungsgemäß bevorzugte Verfahren dadurch gekennzeichnet, daß die Haarbehandlungszusammensetzungen - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat (e) enthalten.

Unter Purin, den Purinen und den Purinderivaten sind erfindungsgemäß einige Vertreter besonders bevorzugt. Erfindungsgemäß bevorzugte Verfahren sind dadurch gekennzeichnet, daß die Haarbehandlungszusammensetzungen als Pflege-Enhancer - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten

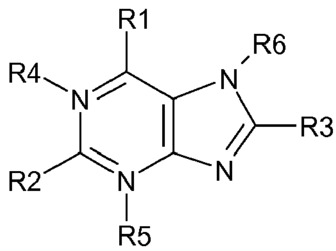

(Pur-I)

in der die Reste $R^1$, $R^2$ und $R^3$ unabhängig voneinander ausgewählt sind aus -H, - OH, $NH_2$, -SH und die Reste $R^4$, $R^5$ und $R^6$ unabhängig voneinander ausgewählt sind aus -H, $-CH_3$ und $-CH_2-CH_3$, wobei folgende Verbindungen bevorzugt sind:

- Purin ($R^1 = R^2 = R^3 = R^4 = R^5 = R^6 = H$)
- Adenin ($R^1 = NH_2$, $R^2 = R^3 = R^4 = R^5 = R^6 = H$)
- Guanin ($R^1 = OH$, $R^2 = NH_2$, $R^3 = R^4 = R^5 = R^6 = H$)
- Harnsäure ($R^1 = R^2 = R^3 = OH$, $R^4 = R^5 = R^6 = H$)
- Hypoxanthin ($R^1 = OH$, $R^2 = R^3 = R^4 = R^5 = R^6 = H$)
- 6-Purinthiol ($R^1 = SH$, $R^2 = R^3 = R^4 = R^5 = R^6 = H$)
- 6-Thioguanin ($R^1 = SH$, $R^2 = NH_2$, $R^3 = R^4 = R^5 = R^6 = H$)
- Xanthin ($R^1 = R^2 = OH$, $R^3 = R^4 = R^5 = R^6 = H$)
- Coffein ($R^1 = R^2 = OH$, $R^3 = H$, $R^4 = R^5 = R^6 = CH_3$)
- Theobromin ($R^1 = R^2 = OH$, $R^3 = R^4 = H$, $R^5 = R^6 = CH_3$)
- Theophyllin ($R^1 = R^2 = OH$, $R^3 = H$, $R^4 = CH_3$, $R^5 = CH_3$, $R^6 = H$).

Es ist weiterhin vorteilhaft, Purin bzw. Purinderivate und Biochinone in einem bestimmten Verhältnis zueinander einzusetzen. Hier sind Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Verfahren sind daher dadurch gekennzeichnet, daß die Haarbehandlungszusammensetzungen - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflege-Enhancer können die im erfindungsgemäßen Verfahren eingesetzten Mittel auch Flavonoide enthalten. Erfindungsgemäß können Flavonoide aus allen sechs Gruppen eingesetzt werden, wobei bestimmte Vertreter

aus den einzelnen Gruppen als Pflege-Enhancer wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Verfahren sind dadurch gekennzeichnet, daß die Haarbehandlungszusammensetzungen - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O*-rutinosid (Rutin), enthalten.

Bevorzugt ist auch der Einsatz von Bisabolol und/oder Bisabololoxiden als Pflege-Enhancer in den erfindungsgemäßen Mitteln. Hier sind erfindungsgemäße Verfahren bevorzugt, bei denen die Haarbehandlungszusammensetzungen zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol

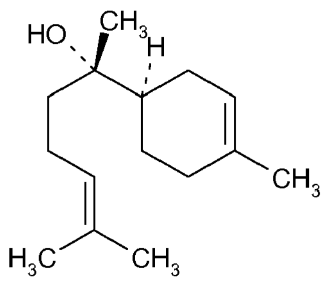

enthalten.

Auch Creatin eignet sich erfindungsgemäß als Pflege-Enhancer. Besonders bevorzugte erfindungsgemäße Verfahren nutzen Haarbehandlungszusammensetzungen, die- bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% *N*-Methyl-guanidino-essigsäure (Creatin) enthalten.

Erfindungsgemäß eingesetzte Zusammensetzungen können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:

Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann.

Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich.

Zur Bekämpfung der hormonellen Einflüße auf die Haarfollikel kann zur äußeren Anwendung Spironolactone in Form von Haarwasser und in Kombination mit Minoxidil angewandt werden. Spironolactone wirkt als Androgen-Rezeptor-Blocker, dh. die Bindung von DHT an die Haarfollikel wird verhindert.

Zusammenfassend sind erfindungsgemäße Verfahren bevorzugt, bei denen die Haarbehandlungszusammensetzungen zusätzlich - bezogen auf ihr Gewicht - 0,001 bis 5 Gew.-% Haarwurzel-stabilisierende Stoffe, insbesondere Minoxidil und/oder Finasterid und/oder Ketoconazol enthalten.

Durch zusätzliche Antischuppenwirkstoffe (beispielsweise Climbazol, Piroctone Olamine oder Zink-Pyrithion) wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, daß die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.

Zusammenfassend sind erfindungsgemäße Verfahren bevorzugt, die Haarbehandlungszusammensetzungen enthalten, die zusätzlich - bezogen auf ihr Gewicht - 0,001 bis 5 Gew.-% Antischuppenwirkstoffe, insbesondere Piroctone Olamine (1-Hydroxy-4-methyl-6-(2,4,4-trirnethylpentyl)pyridin-2(1H)-on, Verbindung mit 2-Aminoethanol, 1:1) und/oder

Zink-Pyrithion und/oder Selensulfid und/oder Climbazol und/oder Salicylsäure oder Fumarsäure enthalten.

Zusätzlich zu den Pflege-Enhancern können die im erfindungsgemäßen Verfahren eingesetzten Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die im erfindungsgemäßen Verfahren eingesetzten Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-trans-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte im erfindungsgemäßen Verfahren eingesetzte Mittel enthalten eine oder mehrere Aminosäuren in engeren Mengenbereichen. Hier sind erfindungsgemäß bevorzugte Verfahren dadurch gekennzeichnet, daß die Mittel als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die im erfindungsgemäßen Verfahren eingesetzten Mittel mindestens ein Kohlenhydrat aus der Gruppe der Monosaccharide, Disaccharide und/oder Oligosaccharide enthalten. Hier sind erfindungsgemäß bevorzugte Verfahren dadurch gekennzeichnet, daß die Haarbehandlungsmittel als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus

- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

Besonders bevorzugte im erfindungsgemäßen Verfahren eingesetzte Mittel enthalten bezogen auf ihr Gewicht

- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Wie bereits erwähnt, enthalten bevorzugte im erfindungsgemäßen Verfahren eingesetzte Mittel (eine) Aminosäure(n).

Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Allün), L-trans-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte im erfindungsgemäßen Verfahren eingesetzte Mittel enthalten eine oder mehrere Aminosäuren in engeren Mengenbereichen. Hier sind erfindungsgemäß bevorzugte Verfahren dadurch gekennzeichnet, daß die Mittel zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte im erfindungsgemäßen Verfahren eingesetzte Mittel enthalten bezogen auf ihr Gewicht

- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,

- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.

Eine besonders bevorzugte Gruppe von Inhaltsstoffen stellen die Silikone dar.

Erfindungsgemäße bevorzugte Verfahren sind dadurch gekennzeichnet, daß die Mittel mindestens ein Silicon, vorzugsweise ein Silicon enthalten, das ausgewählt ist unter:

(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;

(ii) Polysiloxanen, die in ihrer allgemeinen Struktur eine oder mehrere organofunktionelle Gruppen enthalten, die ausgewählt sind unter:

a) substituierten oder unsubstituierten aminierten Gruppen;
b) (per)fluorierten Gruppen;
c) Thiolgruppen;
d) Carboxylatgruppen;
e) hydroxylierten Gruppen;
f) alkoxylierten Gruppen;
g) Acyloxyalkylgruppen;
h) amphoteren Gruppen;
i) Bisulfitgruppen;
j) Hydroxyacylaminogruppen;
k) Carboxygruppen;
l) Sulfonsäuregruppen; und
m) Sulfat- oder Thiosulfatgruppen;

(iii) linearen Polysiloxan(A)- Polyoxyalkylen(B)- Blockcopolymeren vom Typ $(A-B)_n$ mit $n > 3$;

(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;

(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;

oder deren Gemischen.

Im erfindungsgemäßen Verfahren besonders bevorzugte einsetzbare Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte im erfindungsgemäßen Verfahren eingesetzte Mittel sind dadurch gekennzeichnet, daß sie mindestens ein Silikon der Formel Si-I

$$(CH_3)_3Si\text{-}[O\text{-}Si(CH_3)_2]_x\text{-}O\text{-}Si(CH_3)_3 \qquad \text{(Si-I)},$$

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet. Bevorzugte erfindungsgemäß einsetzbare Silikone weisen bei 20°C Viskositäten von 0,2 bis 2 $mm^2s^{-1}$ auf, wobei Silikone mit Viskositäten von 0,5 bis 1 $mm^2s^{-1}$ besonders bevorzugt sind.

Besonders bevorzugte im erfindungsgemäßen Verfahren eingesetzte Mittel enthalten ein oder mehrere aminofunktionelle Silicone. Solche Silicone können z.B. durch die Formel

$$M(R_aQ_bSiO_{(4-a-b)/2)x}(R_cSiO_{(4-c)/2)y}M$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2CH_2CH_2-$, $-CH_2CH(CH_3)C(O)OCH_2-$, $-(CH_2)_3CC(O)OCH_2CH_2-$, $-C_6H_4C_6H_4-$, $-C_6H_4CH_2C_6H_4-$; und $-(CH_2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist $-NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH_2NH_2$-Rest. Eine andere mögliche Formel für Z ist $-N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_b SiO_{(4-a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte im erfindungsgemäßen Verfahren eingesetzte Mittel sind dadurch gekennzeichnet, daß sie ein aminofunktionelles Silikon der Formel (Si-II)

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(Si-II)},$$

enthalten, worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$, $-CH_2CH_2CH_3$, $-O-CH(CH_3)_2$, $-CH(CH_3)_2$, $-O-CH_2CH_2CH_2CH_3$, $-CH_2CH_2CH_2CH_3$, $-O-CH_2CH(CH_3)_2$, $-CH_2CH(CH_3)_2$, $-O-CH(CH_3)CH_2CH_3$, $-CH(CH_3)CH_2CH_3$, $-O-C(CH_3)_3$, $-C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

  o $-Q-N(R'')-CH_2-CH_2-N(R'')_2$
  o $-Q-N(R'')_2$
  o $-Q-N^+(R'')_3A^-$
  o $-Q-N^+H(R'')_2 A^-$
  o $-Q-N^+H_2(R'')A^-$
  o $-Q-N(R'')-CH_2-CH_2-N^+R''H_2A^-$,

  wobei jedes Q für eine chemische Bindung, $-CH_2-$, $-CH_2-CH_2-$, $-CH_2CH_2CH_2-$, $-C(CH_3)_2-$, $-CH_2CH_2CH_2CH_2-$, $-CH_2C(CH_3)_2-$, $-CH(CH_3)CH_2CH_2-$ steht,

R" für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, - $CH(CH_3)_2$, $-CH_2CH_2CH_2H_3$, $-CH_2CH(CH_3)_2$, $-CH(CH_3)CH_2CH_3$, -C $(CH_3)_3$, steht und A ein Anion repräsentiert, welches vorzugsweise ausgewählt ist aus Chlorid, Bromid, Iodid oder Methosulfat.

Besonders bevorzugte im erfindungsgemäßen Verfahren eingesetzte Mittel sind dadurch gekennzeichnet, daß sie mindestens ein aminofunktionelles Silikon der Formel (Si-IIa)

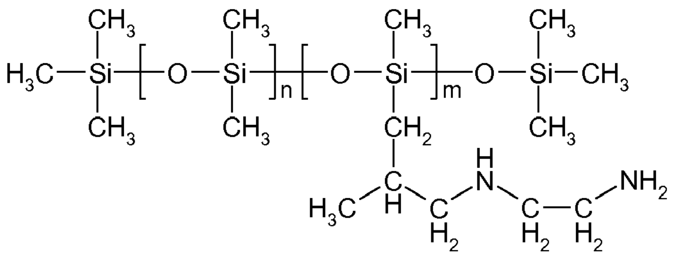

(Si-IIa),

enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch im erfindungsgemäßen Verfahren eingesetzte Mittel, die ein aminofunktionelles Silikon der Formel (Si-IIb)

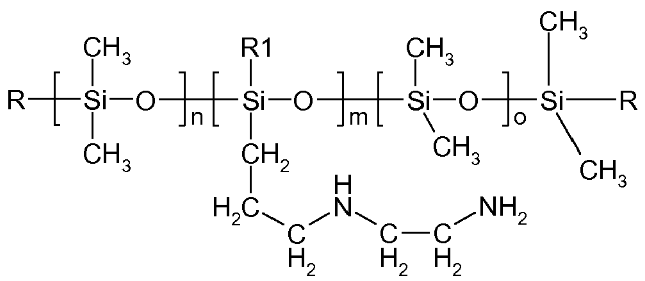

(Si-IIb),

enthalten, worin R für -OH, $-O-CH_3$ oder eine $-CH_3$-Gruppe steht und m, n und o Zahlen sind, deren Summe (m + n + o) zwischen 1 und 2000, vorzugsweise zwischen 50 und 150 beträgt,
wobei die Summe (n + o) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind im erfindungsgemäßen Verfahren eingesetzte Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugt einsetzbare Mittel sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind im erfindungsgemäßen Verfahren eingesetzte Mittel bevorzugt, die mindestens ein Silikon der Formel Si-III

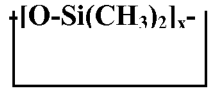

(Si-III)

enthalten, in der x für eine Zahl von 3 bis 200, vorzugsweise von 3 bis 10, weiter bevorzugt von30 bis 7 und insbesondere 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Erfindungsgemäß ebenfalls bevorzugt einsetzbare Mittel sind dadurch gekennzeichnet, daß sie mindestens ein Silikon der Formel Si-IV

$$R_3Si\text{-}[O\text{-}SiR_2]_x\text{-}(CH_2)_n\text{-}[O\text{-}SiR_2]_y\text{-}O\text{-}SiR_3 \qquad \text{(Si-IV)},$$

enthalten, in der R für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2H_3$, $-CH_2CH(CH_3)_2$, $-CH(CH_3)CH_2CH_3$, $-C(CH_3)_3$, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugt einsetzbare Mittel sind dadurch gekennzeichnet, daß sie mindestens ein wasserlösliches Silikon enthalten.

Aus ästhetischen Gründen werden "klare" Produkte von Verbrauchern oft bevorzugt. Erfindungsgemäß bevorzugt einsetzbare Haarbehandlungsmittel sind daher dadurch gekennzeichnet, daß sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibrierten Nephelometer gemessenen Trübung einer Flüssigkeit.

Weiterhin kann in einer bevorzugten Ausführungsform der Erfindung ein im erfindungsgemäßen Verfahren eingesetztes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB (280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul®M 40, Uvasorb®MET, Neo Heliopan®BB, Eusolex®4360), 2-Phenylbenzimidazol-5-sulfonsäure und deren Kalium-, Natrium- und Triethanolaminsalze (Phenylbenzimidazole sulfonic acid; Parsol®HS; Neo Heliopan®Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parso1®1789, Eusolex®9020), α-(2-Oxoborn-3-yliden)-toluol-4-sulfonsäure und deren Salze, ethoxylierte 4-Aminobenzoesäure-ethylester (PEG-25 PABA; Uvinul®P 25), 4-Dimethylaminobenzoesäure-2-ethylhexylester (Octyl Dimethyl PABA; Uvasorb®DMO, Escalol®507, Eusolex®6007), Salicylsäure-2-ethylhexylester (Octyl Salicylat; Escalol®587, Neo Heliopan®OS, Uvinul®O18), 4-Methoxyzimtsäure-isopentylester (Isoamyl p-Methoxycinnamate; Neo Heliopan®E 1000), 4-Methoxyzimtsäure-2-ethylhexyl-ester (Octyl Methoxycinnamate; Parsol®MCX, Escalol®557, Neo Heliopan®AV), 2-Hydroxy-4-methoxybenzophenon-5-sulfonsäure und deren Natriumsalz (Benzophenone-4; Uvinul®MS 40; Uvasorb®S 5), 3-(4'-Methylbenzyliden)-D,L-Campher (4-Methylbenzylidene camphor; Parsol®5000, Eusolex®6300), 3-Benzylidencampher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb®20 H, Uvinul®400), 1,1'-Diphenylacrylonitrilsäure-2-ethylhexyl-ester (Octocrylene; Eusolex®OCR, Neo Heliopan®Type 303, Uvinu®N 539 SG), o-Aminobenzoesäure-menthylester (Menthyl Anthranilate; Neo Heliopan®MA), 2,2',4,4'-Tetrahydroxybenzophenon (Benzophenone-2; Uvinul®D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxy-benzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise substituierte Benzophenone, p-Aminobenzoesäureester, Diphenylacrylsäureester, Zimtsäureester, Salicylsäureester, Benzimidazole und o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur $-(CH_2)_x-N^+R^1R^2R^3\ X^-$, in der x steht für eine ganze Zahl von 1 bis 4, $R^1$ und $R^2$ unabhängig voneinander stehen für $C_{1-4}$-Alkylgruppen, $R^3$ steht für eine $C_{1-22}$-Alkylgruppe oder eine Benzylgruppe und $X^-$ für ein physiologisch verträgliches Anion. Im Rahmen dieser allgemeinen Struktur steht x bevorzugt für die die Zahl 3, $R^1$ und $R^2$ jeweils für eine Methylgruppe und $R^3$ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyl-dimethylaminobenzamidopropyl-dimethylammoniumtosylat (Escalol® HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den im erfindungsgemäßen Verfahren eingesetzten Mitteln üblicherweise in Mengen 0,1-5 Gew.-%, bezogen auf das gesamte Mittel, enthalten. Mengen von 0,4-2,5 Gew.-% sind bevorzugt.

Die im erfindungsgemäßen Verfahren eingesetzten Mittel können weiterhin eine 2-Pyrrolidinon-5-carbonsäure

und deren Derivate (J) enthalten. Bevorzugt sind die Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den im erfindungsgemäßen Verfahren eingesetzten Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Zusätzlich kann es sich als vorteilhaft erweisen, wenn in den im erfindungsgemäßen Verfahren eingesetzten Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß verwendeten Carbonsäuren können selbstverständlich entlang der Kohlenstoffkette oder des Ringgerüstes substituiert sein. Zu den Substituenten der erfindungsgemäß verwendeten Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl-und/oder Alkenylreste weiter substituiert sein kann. Weiterhin sind ebenfalls bevorzugte Carbonsäurederivate die Phosphon- und Phosphatester.

In einer weiteren bevorzugten Ausführungsform können die im erfindungsgemäßen Verfahren eingesetzten Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koalessenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.

- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die im erfindungsgemäßen Verfahren eingesetzten Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn zusätzlich zu dem bzw. den Polymer(en) aus der Gruppe der kationischen und/oder amphoteren Polymere weitere Polymere (G) in den im erfindungsgemäßen Verfahren eingesetzten Mitteln enthalten sind. In einer bevorzugten Ausführungsform werden den im erfindungsgemäßen Verfahren eingesetzten Mitteln daher weitere Polymere zugesetzt, wobei sich sowohl anionische als auch nichtionische Polymere als wirksam erwiesen haben.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren.

Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Die im erfindungsgemäßen Verfahren eingesetzten Mittel können in einer weiteren Ausführungsform nichtionogene Polymere (G4) enthalten.

Geeignete nichtionogene Polymere sind beispielsweise:

- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls

bevorzugte nichtionische Polymere.

- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere (G) sind in den im erfindungsgemäßen Verfahren eingesetzten Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Die im erfindungsgemäßen Verfahren eingesetzten Mittel können weitere Wirk- und Hilfsstoffe beinhalten. Diese werden nachfolgend beschrieben.

Vorzugsweise enthalten die im erfindungsgemäßen Verfahren eingesetzten Mittel zusätzlich mindestens einen Emulgator bzw. ein Tensid, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind.

Erfindungsgemäß bevorzugt einsetzbare Haarbehandlungsmittel sind dadurch gekennzeichnet, daß sie - bezogen auf sein Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthalten.

Als anionische Tenside und Emulgatoren eignen sich für die erfindungsgemäß eingesetzten Zusammensetzungen alle für die Verwendung am menschlichen Körper geeigneten anionischen oberflächenaktiven Stoffe. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I),

$$\begin{array}{c} XOOC\text{-}CH_2CH_2CH\text{-}COOX \\ | \\ HN\text{-}COR^1 \end{array} \qquad (I)$$

in der $R^1CO$ für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise $C_{12/14}$- bzw. $C_{12/18}$-Kokosfettsäure, Laurinsäure, Myristinsäure, Palmitinsäure und/oder Stearinsäure, insbesondere Natrium-N-cocoyl-und Natrium-N-stearoyl-L-glutamat,

- Ester einer hydroxysubstituierten Di- oder Tricarbonsäure der allgemeinen Formel (II),

X

|

HO — C — $COOR^1$ (II)

|

Y — CH — $COOR^2$

in der X=H oder eine $-CH_2COOR$-Gruppe ist, Y=H oder -OH ist unter der Bedingung, dass Y=H ist, wenn X=-$CH_2COOR$ ist, R, $R^1$ und $R^2$ unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten ($C_6$-$C_{18}$)-Alkylpolysaccharide mit 1 bis 6 monomeren Saccharideinheiten und/oder der veretherten aliphatischen ($C_6$-$C_{16}$)-Hydroxyalkylpolyole mit 2 bis 16 Hydroxylresten ausgewählt sind, unter der Maßgabe, dass wenigstens eine der Gruppen R, $R^1$ oder $R^2$ ein Rest Z ist,

- Ester der Sulfobernsteinsäure oder der Sulfosuccinate der allgemeinen Formel (III),

$H_2C$ — $COOR^1$ (III)

|

$M^{(n+/n)}\ {}^{-}O_3S$ — CH — $COOR^2$

in der $M^{(n+/n)}$ für n = 1 ein Wasserstoffatom, ein Alkalimetallkation, eine Ammoniumgruppe oder das Kation einer ammonium-organischen Base und für n = 2 ein Erdalkalimetallkation darstellt und $R^1$ und $R^2$ unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten ($C_6$-$C_{18}$)-Alkylpolysaccharide mit 1 bis 6 monomeren Saccharideinheiten und/oder der veretherten aliphatischen ($C_6$-$C_{16}$)-Hydroxyalkylpolyole mit 2 bis 16 Hydroxylresten ausgewählt ist, unter der Maßgabe, dass wenigstens eine der Gruppen $R^1$ oder $R^2$ ein Rest Z ist,

- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel $R-(O-CH_2-CH_2)_x-OSO_3H$, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an $C_{8-22}$-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Als zwitterionische Tenside und Emulgatoren werden solche oberflächenaktiven Verbindungen bezeichnet, die im Molekül mindestens eine quartäre Ammoniumgruppe und mindestens eine - $COO^{(-)}$ - oder -$SO_3^{(-)}$ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethyl-ammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl-oder Acylgruppe sowie das

Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer $C_8$ - $C_{24}$ - Alkyl- oder -Acylgruppe mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das $C_{12}$ - $C_{18}$ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- $C_{12}$-$C_{30}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen® HSP (Cognis) oder Sovermo® - Typen (Cognis), insbesondere von gesättigten $C_{8-30}$-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid-oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel $RO-(Z)_x$ wobei R für Alkyl, Z für Zucker sowie x für die Anzahl der Zuckereinheiten steht.
  Besonders bevorzugt sind solche Alkylpolyglycoside, bei denen R

  - im wesentlichen aus $C_8$- und $C_{10}$-Alkylgruppen,
  - im wesentlichen aus $C_{12}$- und $C_{14}$-Alkylgruppen,
  - im wesentlichen aus $C_8$- bis $C_{16}$-Alkylgruppen oder
  - im wesentlichen aus $C_{12}$- bis $C_{16}$-Alkylgruppen oder
  - im wesentlichen aus $C_{16}$ bis $C_{18}$-Alkylgruppen

besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

- Gemische aus Alkyl-(oligo)-glucosiden und Fettalkoholen, z. B. Montanov®68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls® PGPH) oder Triglycerindiisostearat (Lameform® TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls

im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Ganz besonders bevorzugt sind im erfindungsgemäßen Verfahren eingesetzte Mittel, die zusätzlich Fettalkohol (e) und/oder Fettalkoholalkoxylat(e), vorzugsweise $C_{12-22}$-Fettalkohol(e) und/oder $C_{12-22}$-Fettalkoholethoxylat(e) mit 10 bis 30 EO-Einheiten, besonders bevorzugt $C_{16-18}$-Fettalkohol(e) und/oder $C_{16-18}$-Fettalkoholethoxylat(e) mit 12 bis 20 EO-Einheiten, vorzugsweise in Mengen von 5 bis 20 Gew.-%, bevorzugt von 7,5 bis 17,5 Gew.-% und insbesondere von 10 bis 15 Gew.-%, jeweils bezogen auf das Gewicht des Mittels, enthalten.

Zusammenfassend sind im erfindungsgemäßen Verfahren eingesetzte Haarbehandlungsmittel bevorzugt, die - bezogen auf ihr Gewicht - 0,1 bis 20 Gew.-%, vorzugsweise 0,25 bis 17,5 Gew.-% und insbesondere 5 bis 15 Gew.-% anionische(s) Tensid(e), besonders bevorzugt Fettalkoholethersulfate der Formel

$$H_3C\text{-}(CH_2)_n\text{-}(OCH_2CH_2)_k\text{-}OSO_3^-\ M^+$$

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe $Na^+$, $K^+$ $NH_4^+$, ½ $Mg^{2+}$, ½ $Zn^{2+}$, vorzugsweise für $Na^+$, stehen.

Bevorzugte im erfindungsgemäßen Verfahren eingesetzte Haarbehandlungsmittel sind weiter dadurch gekennzeichnet, daß sie zusätzlich amphotere(s) Tensid(e), vorzugsweise aus den Gruppen der

- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- $C_{12}$ - $C_{18}$ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen,

enthalten, wobei bevorzugte Mittel das bzw. die amphotere(n) Tensid(e) in Mengen von 1 bis 15 Gew.-%, vorzugsweise von 2,5 bis 12 Gew.-% und insbesondere von 5 bis 10 Gew.-%, jeweils bezogen auf das gesamte Mittel, enthalten.

Im erfindungsgemäßen Verfahren wird die Haarbehandlungszusammensetzung, die mindestens die Inhaltstoffe i, ii und iii enthält, auf trockenes, handtuchtrockenes oder nasses Haar aufgetragen. Bevorzugte erfindungsgemäße Verfahren sind dadurch gekennzeichnet, daß die Haarbehandlungszusammensetzung auf feuchtes oder nasses Haar aufgetragen wird.

Die Applikation bzw. das Auftragen der Haarbehandlungszusammensetzung kann in vielfältiger Weise erfolgen, beispielsweise kann eine fließfähige Zusammensetzung aus einem Behälter auf die Hände oder direkt auf das haar appliziert und dort händisch eingearbeitet werden. Auch die Applikation mittels eine Spray-Spenders oder als Schaum ist erfindungsgemäß möglich.

Nach dem Auftragen der Haarbehandlungszusammensetzung auf die keratinischen Fasern werden diese abgedeckt, so daß keine Komponente der Haarbehandlungszusammensetzung entweichen kann, um eine intensive Wirkung der Zusammensetzung zu gewährleisten. Vorzugsweise erfolgt die Abdeckung durch Tücher oder Hauben, aus Materialien mit kleiner Wasserdampfdurchlässigkeit.

Besonders bevorzugte erfindungsgemäße Verfahren sind dadurch gekennzeichnet, daß die mit der Haarbehandlungszusammensetzung beaufschlagten Haare mit einer Haube abgedeckt werden, welche aus synthetischen Polymeren gefertigt ist.

Ganz besonders bevorzugte Haubenmaterialien sind solche, die eine Feuchtigkeitsdampfdurchlässigkeitsrate von 0,1 g/m$^2$/Tag bis weniger als 20 g/m$^2$/Tag aufweisen, wenn das Material bei 23°C und einer relativen Gleichgewichtsfeuchtigkeit von 85% gelagert wird. Die genannten Temperatur- und Feuchtigkeitsbedingungen sind die Prüfbedingungen, die in der DIN-Norm 53122 genannt werden, wobei laut DIN 53122 minimale Abweichungen zulässig sind (23 $\pm$ 1°C, 85 $\pm$ 2% rel. Feuchte). Die Feuchtigkeitsdampfdurchlässigkeitsrate eines gegebenen Haubenmaterials läßt sich nach weiteren Standardmethoden bestimmen und ist beispielsweise auch im ASTM-Standard E-96-53T ("Test for measuring Water Vapor transmission of Materials in Sheet form") und im TAPPI Standard T464 m-45 ("Water Vapor Permeability of Sheet Materials at high temperature an Humidity") beschrieben. Das Meßprinzip gängiger Verfahren beruht dabei auf der Wasseraufnahme von wasserfreiem Calciumchlorid, welches in einem Behälter in der entsprechenden Atmosphäre gelagert wird, wobei der Behälter an der Oberseite mit dem zu testenden Material verschlossen ist. Aus der Oberfläche des Behälters, die mit dem zu testenden Material verschlossen ist (Permeationsfläche), der Gewichtszunahme des Calciumchlorids und der Expositionszeit läßt sich die Feuchtigkeitsdampfdurchlässigkeitsrate nach

$$FDDR = \frac{24 \cdot 10000}{A} \cdot \frac{x}{y} \left[ g / m^2 / 24h \right]$$

berechnen, wobei A die Fläche des zu testenden Materials in cm$^2$, x die Gewichtszunahme des Calciumchlorids in g und y die Expositionszeit in h bedeutet.

Die relative Gleichgewichtsfeuchtigkeit, oft als "relative Luftfeuchtigkeit" bezeichnet, beträgt bei der Messung der Feuchtigkeitsdampfdurchlässigkeitsrate im Rahmen der vorliegenden Erfindung 85% bei 23°C. Die Aufnahmefähigkeit von Luft für Wasserdampf steigt mit der Temperatur bis zu einem jeweiligen Höchstgehalt, dem sogenannten Sättigungsgehalt, an und wird in g/m$^3$ angegeben. So ist beispielsweise 1 m$^3$ Luft von 17° mit 14,4 g Wasserdampf gesättigt, bei einer Temperatur von 11° liegt eine Sättigung schon mit 10 g Wasserdampf vor. Die relative Luftfeuchtigkeit ist das in Prozent ausgedrückte Verhältnis des tatsächlich vorhandenen Wasserdampf-Gehalts zu dem der herrschenden Temperatur entsprechenden Sättigungs-Gehalt. Enthält beispielsweise Luft von 17° 12 g/m$^3$ Wasserdampf, dann ist die relative Luftfeuchtigkeit = (12/14,4)·100 = 83%. Kühlt man diese Luft ab, dann wird die Sättigung (100% r. L.) beim sogenannten Taupunkt (im Beispiel: 14°) erreicht, d.h., bei weiterem Abkühlen bildet sich ein Niederschlag in Form von Nebel (Tau). Zur quantitativen Bestimmung der Feuchtigkeit benutzt man Hygrometer und Psychrometer.

Die relative Gleichgewichtsfeuchtigkeit von 85% bei 23°C läßt sich beispielsweise in Laborkammern mit Feuchtigkeitskontrolle je nach Gerätetyp auf +/- 2% r.L. genau einstellen. Auch über gesättigten Lösungen bestimmter Salze bilden sich in geschlossenen Systemen bei gegebener Temperatur konstante und wohldefinierte relative Luftfeuchtigkeiten aus, die auf dem Phasen-Gleichgewicht zwischen Partialdruck des Wassers, gesättigter Lösung und Bodenkörper beruhen.

Im Rahmen der vorliegenden Erfindung bevorzugte Haubenmaterialien weisen eine Feuchtigkeitsdampfdurchlässigkeitsrate von 0,5 g/m$^2$/Tag bis weniger als 15 g/m$^2$/Tag auf.

Die mit der Haarbehandlungszusammensetzung beaufschlagten und abgedeckten Haare werden im erfindungsgemäßen Verfahren dann eine Zeitlang abgedeckt belassen, um eine optimale Einwirkung der Haarbehandlungszusammensetzung zu gewährleisten. Es hat sich herausgestellt, daß der Zeitraum von einer halben Minute bis zu zehn Minuten hierfür optimal ist, wobei in bevorzugten erfindungsgemäßen Verfahren die Haarbehandlungszusammensetzung über einen Zeitraum von 45 bis 450 Sekunden, vorzugsweise von 60 bis 360 Sekunden, besonders bevorzugt von 90 bis 300 Sekunden und insbesondere von 120 bis 240 Sekunden einwirken gelassen wird.

Die Einwirkung kann durch leicht erhöhte Temperatur verbessert werden. Entgegen der allgemeinen Annahmen, daß der Penetrationsprozeß umso besser verläuft, je höher die Temperatur ist, hat sich herausgestellt, daß ein Temperaturoptimum um die 40°C herum existiert, Temperaturen über 55-60°C verschlechtern das Ergebnis ebenso wie Temperaturen unter 20-25°C. In bevorzugten erfindungsgemäßen Verfahren werden die abgedeckten Haare zwischen den Schritten c) und d) daher auf Temperaturen zwischen 25 und 55°C, vorzugsweise 30 und 52,5°C, besonders bevorzugt zwischen 35 und 50°C und insbesondere zwischen 37 und 45°C erwärmt.

Im Anschluß an die - ggf. durch Erwärmung unterstützte - Einwirkzeit werden die Haare von der Abdeckung befreit und die Haarbehandlungszusammensetzung auf herkömmliche Art ausgewaschen. Danach kann das Haar gewünschtenfalls mit einem Haartrockner getrocknet oder frisiert werden.

Im Vergleich zur herkömmlichen Vorgehensweise werden der Griff des trockenen und nassen Haares sowie das Volumen der Frisur deutlich verbessert. Dieser Effekt hält ebenfalls im Gegensatz zu herkömmlichen Vorgehensweisen über mehrere Haarwäschen hinweg an, ohne die Haar fettig oder unnötig schwer erscheinen zu lassen.

**Patentansprüche**

**1.** Verfahren zur kosmetischen Behandlung von Haar, umfassend die Schritte

a) Auftragen einer Haarbehandlungszusammensetzung, die

i. mindestens eine kationische Verbindung der Formel (I)

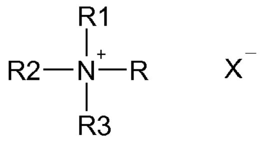

(I),

in der

- die Reste R, R1, R2 unabhängig voneinander für einen gegebenenfalls substituierten Alkyl- oder Alkenyl- oder Acylrest stehen, wobei
- R1 und R2 miteinander einen Ring bilden können,
- R3 für-$CH_3$ oder -$CH_2CH_3$ steht, und
- $X^-$ für ein Anion aus der Gruppe Chlorid, Bromid, Methosulfat steht,

ii. mindestens einen Pflegestoff,
iii. mindestens 30 Gew.-% Wasser

enthält,
auf trockenes oder handtuchtrockenes oder nasses Haar,
b) Abdeckung der Haare, daß keine Komponente der Haarbehandlungszusammensetzung entweichen kann
c) Einwirkenlassen der Zusammensetzung über einen Zeitraum von 30 bis 600 Sekunden,
d) Abnehmen der Abdeckung und Auswaschen der Haarbehandlungszusammensetzung.

**2.** Verfahren nach Anspruch 1, **dadurch gekennzeichnet, daß** das Haarbehandlungsmittel - bezogen auf sein Gewicht - 0,25 bis 10 Gew.-%, vorzugsweise 0,5 bis 7,5 Gew.-%, weiter bevorzugt 0,75 bis 5 Gew.-% und insbesondere 1 bis 3,5 Gew.-% kationische Verbindung(en) der Formel (I) enthält.

**3.** Verfahren nach einem der Ansprüche 1 oder 2, **dadurch gekennzeichnet, daß** die Haarbehandlungszusammensetzung mindestens eine kationische Verbindung der Formel (Ia) enthält

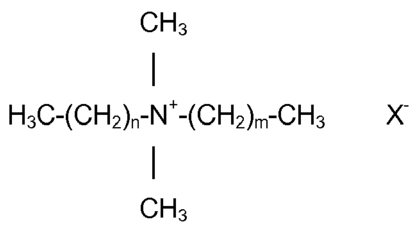

(Ia),

in der n und m unabhängig voneinander für ganze Zahlen zwischen 5 und 21 stehen, wobei bevorzugte Werte für n und m die Zahlen 7, 9, 11, 13, 15 und 17 sind und bevorzugt n = m = 17 gilt und $X^-$ für ein Anion aus der Gruppe Chlorid, Bromid, Methosulfat steht.

**4.** Verfahren nach einem der Ansprüche 1 bis 3, **dadurch gekennzeichnet, daß** die Haarbehandlungszusammensetzung mindestens eine kationische Verbindung der Formel (Ib) enthält

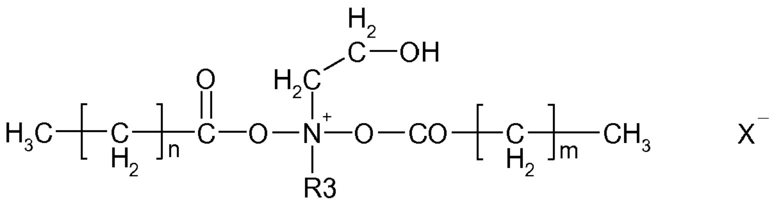

(Ib),

in der R3 für $-CH_3$ oder $-CH_2CH_3$ steht, n und m unabhängig voneinander für ganze Zahlen zwischen 4 und 20 stehen, wobei bevorzugte Werte für n und m die Zahlen 6, 8, 10, 12, 14 und 16 sind und bevorzugt n = m = 16 gilt und $X^-$ für ein Anion aus der Gruppe Chlorid, Bromid, Methosulfat steht.

**5.** Verfahren nach einem der Ansprüche 1 bis 4, **dadurch gekennzeichnet, daß** die Haarbehandlungszusammensetzung mindestens eine kationische Verbindung der Formel (Ic) enthält

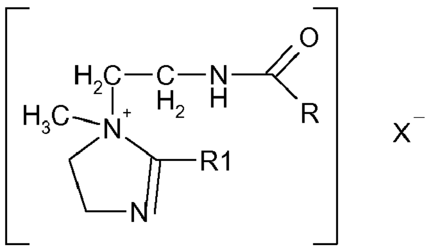

(Ic),

in der R für einen linearen Alkyl- oder Alkenylrest mit 7 bis 21 C-Atomen und R1 für -H oder - $CH_3$ oder $-CH_2CH_3$ oder $-CH(CH_3)_2$ oder $-CH_2CH_2CH_3$ und $X^-$ für ein Anion aus der Gruppe Chlorid, Bromid, Methosulfat steht.

**6.** Verfahren nach einem der Ansprüche 1 bis 5, **dadurch gekennzeichnet, daß** die Haarbehandlungszusammensetzung mindestens einen Pflegestoff aus der Gruppe

i. L-Carnitin und/oder seiner Salze;
ii. Panthenol und/oder Panthothensäure;
iii. der 2-Furanone und/oder deren Derivate, insbesondere Pantolacton;
iv. Taurin und/oder seiner Salze;
v. Niacinamid;
vi. Ubichinon
vii. Ectoin;
viii. Allantoin;
ix. Extrakten von Echinacea

enthält.

**7.** Verfahren nach einem der Ansprüche 1 bis 6, **dadurch gekennzeichnet, daß** die Haarbehandlungszusammensetzung auf feuchtes oder nasses Haar aufgetragen wird.

**8.** Verfahren nach einem der Ansprüche 1 bis 7, **dadurch gekennzeichnet, daß** die mit der Haarbehandlungszusammensetzung beaufschlagten Haare mit einer Haube abgedeckt werden, welche aus synthetischen Polymeren gefertigt ist.

**9.** Verfahren nach einem der Ansprüche 1 bis 8, **dadurch gekennzeichnet, daß** die Haarbehandlungszusammensetzung über einen Zeitraum von 45 bis 450 Sekunden, vorzugsweise von 60 bis 360 Sekunden, besonders bevorzugt von 90 bis 300 Sekunden und insbesondere von 120 bis 240 Sekunden einwirken gelassen wird.

**10.** Verfahren nach einem der Ansprüche 1 bis 9, **dadurch gekennzeichnet, daß** die abgedeckten Haare zwischen den Schritten c) und d) auf Temperaturen zwischen 25 und 55°C, vorzugsweise 30 und 52,5°C, besonders bevorzugt zwischen 35 und 50°C und insbesondere zwischen 37 und 45°C erwärmt werden.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1147762 A, Kao
- WO 9213829 A
- DE 3725030 A